# EUROPEAN PATENT SPECIFICATION

(11) **EP 0 696 936 B1**
(45) Date of publication and mention of the grant of the patent: **14.05.1997**
(21) Application number: 94916600.3
(22) Date of filing: 29.04.1994
(51) Int. Cl.: B01J 23/78, B01J 27/232, C07C 5/333

(54) **DEHYDROGENATION CATALYST HAVING IMPROVED MOISTURE STABILITY, AND PROCESS FOR MAKING AND USING THE CATALYST**
DEHYDRIERUNGKATALYSATOR MIT EINER VERBESSERTEN FEUCHTIGKEITBESTÄNDIGKEIT,SOWIE VERFAHREN ZU DESSEN HERSTELLUNG UND DESSEN VERWENDUNG
CATALYSEUR DE DESHYDROGENATION PRESENTANT UNE STABILITE A L'HUMIDITE AMELIOREE, SON PROCEDE DE FABRICATION ET D'UTILISATION

(30) Priority: 04.05.1993 US 58192
(43) Date of publication of application: 21.02.1996
(73) Proprietor: THE DOW CHEMICAL COMPANY, Midland, Michigan 48640 (US)
(72) Inventor: DELLINGER, Phillip, W., Lake Jackson, TX 77566 (US); MOORE, Rebecca, G., Lake Jackson, TX 77566 (US); SHERROD, Fred, A., Lake Jackson, TX 77566 (US); SMITH, Allen, R., Lake Jackson, TX 77566 (US)
(74) Representative: Smulders, Theodorus A.H.J., Ir.
(86) International application number: PCT/US94/04714
(87) International publication number: WO 94/25154

(56) References cited:
- EP-A- 0 181 999
- EP-A- 0 195 252
- EP-A- 0 305 020
- WO-A-90/06907

## Description

This invention relates to improved catalysts for the dehydrogenation of hydrocarbons to a method of making such catalyst compositions, and to use of such catalysts. The catalysts of this invention exhibit improved moisture stability when subjected both to contact with hydrogen and steam at elevated temperatures during the dehydrogenation process and to contact with moisture at a temperature less than 100°C. This improved stability is evidenced by improved crush strength and resistance to swelling and cracking.

Catalytic dehydrogenation of hydrocarbons using various catalyst compositions has been known from just prior to World War II. Commercial examples are the manufacture of styrene and butadiene from ethylbenzene and butylene. Promoted iron oxide catalysts have been found to be especially useful in the dehydrogenation of alkyl aromatic hydrocarbons to vinyl aromatic hydrocarbons. Most commercial iron oxide dehydrogenation catalysts include minor amounts of promoters, for example, salts or oxides of chromium, manganese, bismuth, tungsten, or molybdenum, with chromium being preferred, together with a compound of potassium, for example, potassium oxide or carbonate. The potassium compound gives the catalyst a self-regenerative property that prolongs its useful life for long periods of time without significant loss of activity. Recent improvements include the incorporation of minor amounts of vanadium and modifiers, such as carbon black or graphite and methyl cellulose, which can beneficially affect the pore structures of the catalysts.

The catalyst life of dehydrogenation catalysts is often dictated by the pressure drop across a reactor. An increase in the pressure drop lowers both the yield and conversion to the desired product. Physical degradation of the catalyst typically increases the pressure drop across the reactor. For this reason, the physical integrity of the catalyst is of major importance. Dehydrogenation catalysts containing iron oxide can undergo substantial changes under process conditions which decrease their physical integrity. For example, in the dehydrogenation of ethylbenzene to styrene, the catalyst is subjected to contact with hydrogen and steam at high temperatures (for example, 500°C to 700°C, more typically 540°C to 650°C) and, under these conditions, Fe₂O₃, the preferred source of iron for the production of styrene catalyst, is reduced to Fe₃O₄. This reduction causes a transformation in the lattice structure of the iron oxide, resulting in catalyst bodies which have poorer physical integrity and are very susceptible to degradation by contact with water at temperatures below 100°C. This degradation by contact with water is characterized by the catalyst bodies (for example, pellets or granules) becoming soft and/or swollen and/or cracked. The water which contacts the catalyst may be in the form of liquid or a wet gas, such as air with a high humidity. "High humidity" refers to a relative humidity above 50 percent.

The catalysts in styrene production plants are often exposed to temperatures below 100°C during start-ups, shutdowns, and upsets. Because large amounts of steam are used in styrene production, there is significant potential for exposing the catalyst to moisture at low temperatures.

As previously discussed, this exposure causes physical degradation of the catalyst, which increases pressure drop across the reactor, resulting in decreased catalyst life.

Williams et al., US 5,023,225-A assigned to United Catalysts Inc., teach a predominately iron oxide, chromium oxide modified dehydrogenation catalyst made by first forming a blend (or coprecipitate) of chromium oxide or a salt of chromium and a yellow iron hydrate and heating the blend to convert the yellow iron hydrate to red iron oxide, thereby forming a chromium oxide modified iron oxide precursor. The precursor is then blended with four additional components and calcined to form the catalyst. The four additional components are:
(a) alkali metal hydroxides, carbonates, or bicarbonates, preferably alkali metal oxides, that is, sodium or potassium oxide, with potassium oxide being most preferred, in an amount of from 5 to 15 percent by weight of the catalyst;
(b) alkaline earth metal oxides or hydroxides, preferably alkaline earth metal oxides, that is, magnesium oxide, calcium oxide, or strontium oxide, with both MgO and CaO being preferred, in an amount of from 2 to 10 percent by weight of the catalyst;
(c) oxides and salts of the cerium subgroup having atomic numbers of 57 to 62, preferably oxides of the cerium subgroup, with cerium oxide being preferred, in an amount of from 2 to 10 percent by weight of the catalyst; and
(d) molybdenum or tungsten oxides and salts, preferably oxides of molybdenum or tungsten, with molybdenum oxide being preferred, in an amount of from 1 to 5 percent by weight of the catalyst.

Catalysts with higher amounts of potassium have been used: Chu, US 4,503,163-A assigned to Mobil Oil Company, for example, discloses catalysts for the production of p-methylstyrene from p-ethyltoluene which contain, by weight, 30 to 60 percent, preferably 35 to 55 percent iron oxide calculated as ferric oxide; 13 to 48 percent, preferably 27 to 41 percent, a potassium promoter compound, calculated as potassium oxide; 0 to 5 percent, preferably 1 to 4 percent a chromium compound, calculated as chromic oxide; and 1 to 15 percent, preferably 2 to 10 percent a calcium compound, calculated as CaO. Such catalysts are self regenerative catalysts which perform well at lower steam to oil ratios; for example, ratios of less than 2:1 (by weight). The economic advantages of using less steam are obvious. The problem with using higher concentrations of potassium is that the vulnerability of the used iron oxide catalyst to moisture increases with increasing potassium concentration.

Patent documents EP-A-0 305 020, EP-A-0 195 252 and EP-A-0 181 999 teach catalysts containing components (a), (b), (c) and (e) below as well as calcium present as the carbonate, sulphate or hydroxide.

O'Hara, US 3,904,552-A assigned to Girdler Chemical Inc., teaches a dehydrogenation catalyst consisting essentially of no chromium oxide and containing
(a) iron oxide, from 30 to 90 percent by weight;
(b) a water gas promoter--preferably, and in the Examples exclusively, potassium carbonate--selected from the oxides, hydroxides, carbonates, and bicarbonates, of potassium, cesium, rubidium and sodium, in amounts of from 1 to 40 percent;
(c) cerium oxide, from 0.5 to 10 percent by weight.
(d) molybdenum oxide, from 0.5 to 5 percent by weight, and
(e) an hydraulic cement, such as a Portland cement which, after calcination, contains calcium oxide or another compound not chemically combined with aluminum or silicon compounds and which is therefore available to exert an effect upon the physical properties of the iron oxide, in an amount of from 5 to 30 percent by weight of the total weight of the catalyst.

Turley et al., US 3,703,593-A assigned to The Dow Chemical Company discloses preparation of a dehydrogenation catalyst prepared from a paste containing a mixture of hydrates (yellow) iron oxide and anhydrous (red) iron oxide in particular ratios that is. 0.25:1 to 0.85:1 and 1.86:1 to 4:1 by weight, as the iron oxide component, together with an oxide of an alkali metal, preferably potassium oxide or carbonate, chromium oxide or an alkali metal chromate or dichromate, a refractory cement binder (preferably 2.6 to 3 percent by weight of the paste), and preferably, 5 to 15 weight percent methyl cellulose, and 5 to 15 weight percent graphite. Turley et al. teach their calcined catalyst contained, by weight, 50 to 90 percent Fe₂O₃, 0.5 to 5 percent Cr₂O₅, and 9 to 18 percent K₂O. Moore, US 4,684,619-A assigned to The Dow Chemical Company, teaches preparing a dehydrogenation catalyst having improved moisture stability by calcining conventional catalyst components in two steps. The first step is a partial calcination at 250°C to 600°C for a time sufficient to oxidize and remove porosity controlling organic materials, for example 30 minutes to 4 hours depending on temperature. The second step is carried out at 700°C to 800°C for a time sufficient to convert carbonates present to the corresponding oxides, for example 10 to 60 minutes depending on the amount of CO₂ evolved and its rate of removal.

A need exists for a dehydrogenation catalyst that has both high activity, selectivity and resistance to moisture. A way has now been discovered to enhance moisture resistance of these catalysts without any significant detrimental effects to catalyst performance.

In one embodiment the present invention is a novel calcined dehydrogenation catalyst comprising
(a) at least one iron oxide;
(b) at least one carbonate, bicarbonate, oxide or hydroxide of potassium and/or cesium;
(c) an oxide, carbonate, nitrate, or hydroxide of cerium, or a mixture of at least two thereof;
(d) an hydraulic cement;
(e) an hydroxide, carbonate, bicarbonate, acetate, oxalate, nitrate, or sulfate of sodium, in an amount sufficient to provide from 0.2 to 10 percent sodium, calculated as sodium oxide, by weight of the calcined catalyst; and
(f) a carbonate, sulfate, or hydroxide, of calcium or a mixture of at least two thereof, in an amount sufficient to provide from 1.5 to 20 percent calcium, calculated as calcium oxide, by weight of the calcined catalyst.

In a second embodiment, the invention is a process for preparing such improved dehydrogenation catalysts. This process comprises the steps of
(A) preparing an extrudable mixture by admixing
   (a) at least one iron oxide;
   (b) a carbonate, bicarbonate, oxide or hydroxide of potassium or cesium, or a mixture of at least two thereof;
   (c) an oxide, carbonate, nitrate, or hydroxide of cerium, or a mixture of at least two thereof; and
   (d) an hydraulic cement; with sufficient water to form an extrudable mixture;
(B) forming the extrudable mixture into pellets; and
(C) calcining the pellets into a finished catalyst, characterized by including in the extrudable mixture,
   (e) an hydroxide, carbonate, bicarbonate, acetate, oxalate, nitrate, or sulfate of sodium, in an amount sufficient to provide from 0.2 to 10 percent sodium, calculated as sodium oxide, by weight of the calcined catalyst; and
   (f) a carbonate, sulfate, or hydroxide, of calcium or a mixture of at least two thereof, in an amount sufficient to provide from 1.5 to 20 percent calcium, calculated as calcium oxide, by weight of the calcined catalyst.

The new catalyst compositions are useful for the dehydrogenation of an alkyl aromatic compound to form a vinyl aromatic compound by contacting the alkyl aromatic compound with the dehydrogenation catalyst under dehydrogenating conditions. The new catalysts have improved moisture stability, as evidenced by improved crush strength, which decreases catalyst degradation during process upsets.

The invention resides in the discovery that the addition of sodium and calcium compounds to known dehydrogenation catalysts produce new dehydrogenation catalysts having improved stability. Thus, any of the known class of dehydrogenation catalyst compositions containing red or yellow iron oxides and various catalyst promoters (as disclosed, for example, in US 4,503,163; 3,703,593; and 4,684,619 may be used herein. Iron is generally added to the catalyst compositions of the invention as red iron oxide, Fe₂O₃, or yellow iron oxide, Fe₂O₃·H₂O. Particularly suited are pigment grades of red and yellow iron oxides. Likewise the catalyst promoter can be any potassium or cesium carbonate, bicarbonate, oxide or hydroxide or any mixture thereof taught by the art, that is converted to the corresponding oxide or oxides under calcination conditions. Potassium compounds are the preferred promoters. Potassium carbonate or a mixture of potassium carbonate with potassium oxide is most preferred.

The catalyst compositions of the present invention also may contain, and preferably do contain, cerium to enhance selectivity. Cerium, if used in the catalyst compositions of the present invention, can be added to the catalyst in the form of cerium oxide or in the form of other cerium compounds that decompose upon calcination to form cerium oxide, as for example, cerium carbonate, cerium nitrate, cerium hydroxide or any combination thereof.

The physical strength, activity and selectivity of the catalyst compositions of the present invention can be improved by adding certain binding agents. Binding agents can include and consist of hydraulic cements, for example, calcium aluminate or Portland cement. These cements can be added individually or in combination.

The amount of sodium in the new catalyst, measured as sodium oxide and based on the weight of the calcined catalyst may range from 0.2 to 10 percent. Preferably sodium is present in the catalyst in the range of 0.5 to 5 percent. Most preferably, sodium is present in amounts of from 0.8 to 3 percent. The sodium may be added to the catalyst mixture as sodium hydroxide or carbonate or bicarbonate or other salts such as acetate, oxalate, nitrate, or sulfate.

The amount of calcium in the new catalyst, measured as calcium oxide and based on the weight of the calcined catalyst, may range from 1.5 to 20 percent. Preferably calcium is present in the range of 3 to 15 percent. Most preferably calcium is present in amounts from 4 to 12 percent. Calcium can be added to the catalyst mixture in the form of calcium carbonate, calcium sulfate, calcium hydroxide, or other salts.

Other known catalyst additives can be included in the catalysts of the invention, but are not essential. A chromium compound which can serve as a stabilizer for the active catalytic components is illustrative of an optional but preferred additive. Chromium compounds have previously been added to alkali-promoted iron oxide catalysts to extend their life. Chromium, as used in the compositions of this invention, can be added to the catalyst in the form of a chromium oxide or in the form of chromium compounds which decompose upon calcination to chromium oxides.

Another optional component, used to improve the selectivity of the catalyst, is molybdenum which can be added as its oxide or as a molybdate. Other metal compounds that may be added as promoters include compounds of aluminum, vanadium, cobalt, cadmium, copper, magnesium, manganese, and nickel, providing they can be calcined to the corresponding metal oxide.

The density of the catalyst composition can be modified by the addition of various filler substances, for example, combustible materials such as graphite and methyl cellulose. Such materials can be added to the compositions during preparation, but are burned out after the catalyst pellets have been formed during the calcining step. Porosity promoting aids can also facilitate extrusion of catalyst pellets.

The catalyst components can be mixed in various ways known to the art. One method comprises ballmilling together a mixture of desired compounds, adding a small amount of water, and extruding the composite to produce small pellets, which are then dried and calcined. Another method is mixing the components together with water, drying them to form a powder and tabletizing and calcining the tablets. Another procedure involves mixing the components together with an excess of water, partially drying, and then subsequently extruding, drying, and calcining the resulting pellets. The choice of the mixing method depends on the preference of the skilled artisan.

A preferred method of preparing the catalysts is to blend the catalyst ingredients together, including the ingredients of the present invention, in the presence of sufficient water to make a moist extrudable mixture. This mixture is then extruded to produce extrudates between 3.175 mm (1/8-inch) and 6.35 mm (1/4-inch) in diameter. The extrudates are then calcined under conventional calcining conditions. Calcination temperatures can range from 500°C to 900°C, preferably from 600°C to 800°C. After calcination the extrudates are ready for use as catalysts.

The amounts of components (a)-(d) employed, as well as the amounts, if any, of optional components described herein, are not critical so long as they are present in ranges and relative proportions known in the art, which persons skilled in the art will be able to optimize readily to provide practical dehydrogenation catalysts the moisture stability of which can be improved based on the teachings herein.

### Example

An extrudable mixture in the following examples were prepared from commercially available chemicals. LUMNITE is the trade name for calcium aluminate cement manufactured by Lehigh Cement Company.

An extrudable mixture was made by blending in a heated steam jacketed blade mixer 135 grams(g) red iron oxide (Fe₂O₃), 153.4 g yellow iron oxide (Fe₂O₃·H₂O), 120 g calcium aluminate cement (LUMNITE®), 38 g gypsum (CaSO₄·2H₂O), 120 g calcium carbonate (CaCO₃), 10 g molybdenum oxide (MoO₃), 190.5 g hydrated cerium carbonate (Ce₂(CO₃)₃·5H₂O), 450 g potassium carbonate (K₂CO₃), 10 g potassium dichromate (K₂Cr₂O₇), and 41.5 g of an aqueous 50 percent solution of sodium hydroxide (NaOH). 15 percent (wt) of water was then blended into the formulation. The mixture was mixed and dried in the heated steam jacketed mixer until the formula reaches a consistency suitable for extrusion. The hot extrudable mixture was transferred to a California Model CL-3 laboratory Pellet Mill and extruded (4 mm (5/32 inch) in diameter and 8 mm (10/32 inch) in length).

Four additional extrudates formulations were prepared according to the above procedure, but with different amounts of ingredients. Table 1 lists the formulations of Examples 1-5. The extrudates of Examples 1-5 were then calcined by slowly ramping the temperature to 775°C over a period of two hours, and maintaining at 775°C for 30 minutes. The finished compositions of the calcined catalysts, expressed as shown, are given in Table 2.

**TABLE 1**

| Extrudate Formulation | | | | | |
|---|---|---|---|---|---|
| Component* | Ex. 1 | Ex. 2 | Ex. 3 | Ex. 4 | Ex. 5 |
| Fe₂0₃ | 135 | 135 | 135 | 135 | 135 |
| Fe₂O₃·H₂O | 153.4 | 153.4 | 153.4 | 153.4 | 153.4 |
| LUMNITE | 120 | 120 | 120 | 120 | 120 |
| MoO₃ | 10 | 10 | 10 | 10 | 10 |
| Ce₂(CO3)₃·5H₂O | 190.5 | 190.5 | 190.5 | 190.5 | 190.5 |
| K₂CO₃ | 450 | 450 | 450 | 450 | 450 |
| K₂Cr₂O₇ | 10 | 10 | 10 | 10 | 10 |
| CaSO₄·2H₂O | 38 | 38 | 38 | 38 | 38 |
| CaCO₃ | 120 | 240 | 60 | 60 | 60 |
| NaOH, 50 percent | 41.5 | 45.8 | 19.5 | 39.3 | 66.2 |

| | | | | | |
|---|---|---|---|---|---|
| *All weights in grams | | | | | |

**TABLE 2**

| Components of Calcined Catalyst | | | | | |
|---|---|---|---|---|---|
| Component* | Ex. 1 | Ex. 2 | Ex. 3 | Ex. 4 | Ex. 5 |
| Fe₂0₃ | 23.46 | 21.21 | 25.00 | 24.78 | 24.48 |
| LUMNITE | 10.43 | 9.43 | 11.11 | 11.01 | 10.88 |
| MoO₃ | 0.87 | 0.79 | 0.93 | 0.92 | 0.91 |
| Ce₂O₃ | 10.43 | 9.43 | 11.11 | 11.01 | 10.88 |
| K₂CO₃ | 39.10 | 35.35 | 41.68 | 41.30 | 40.79 |
| K₂Cr₂O₇ | 0.87 | 0.79 | 0.93 | 0.92 | 0.91 |
| CaSO₄ | 2.61 | 2.36 | 2.78 | 2.75 | 2.72 |
| CaCO₃ | 10.43 | 18.85 | 5.56 | 5.51 | 5.44 |
| NaOH | 1.80 | 1.80 | 0.90 | 1.80 | 3.00 |

| | | | | | |
|---|---|---|---|---|---|
| *Weight percent | | | | | |

### Comparative Runs 1 to 4

The same procedure employed in Examples 1-5 was used to make all the comparative catalysts. Table 3 lists the formulations of the extrudates for the comparative catalysts.

**Table 3**

| Comparative Extrudate Formulation | | | | |
|---|---|---|---|---|
| Component* | Comp. Run 1 | Comp. Run 2 | Comp. Run 3 | Comp. Run 4 |
| FE₂0₃ | 135 | 135 | 135 | 135 |
| Fe₂O₃·H₂O | 153.4 | 153.4 | 153.4 | 153.4 |
| LUMNITE | 120 | 120 | 120 | 120 |
| MoO₃ | 10 | 10 | 10 | 10 |
| Ce₂(CO3)₃·5H₂O | 190.5 | 190.5 | 190.5 | 190.5 |
| K₂CO₃ | 450 | 450 | 450 | 450 |
| K₂Cr₂O₇ | 10 | 10 | 10 | 10 |
| CaSO₄·2H₂O | 38 | 38 | -- | 38 |
| CaCO₃ | -- | 60 | -- | -- |
| NaOH, 50 percent | -- | -- | 36 | 39.3 |

| | | | | |
|---|---|---|---|---|
| *All weights in grams | | | | |

The finished compositions of the calcined comparative catalysts, expressed as shown, are given in Table 4.

**TABLE 4**

| Components of Calcined Comparative Catalysts | | | | |
|---|---|---|---|---|
| Component* | Comp. Run 1 | Comp. Run 2 | Comp. Run 3 | Comp. Run 4 |
| Fe₂0₃ | 26.74 | 25.24 | 27.06 | 26.22 |
| LUMNITE | 11.88 | 11.21 | 12.02 | 11.65 |
| MoO₃ | 0.99 | 0.93 | 1.00 | 0.97 |
| Ce₂O₃ | 11.88 | 11.22 | 12.03 | 11.66 |
| K₂CO₃ | 44.55 | 42.05 | 45.09 | 43.70 |
| K₂Cr₂O₇ | 0.99 | 0.93 | 1.00 | 0.97 |
| CaSO₄ | 2.97 | 2.81 | -- | 2.92 |
| CaCO₃ | -- | 5.62 | -- | -- |
| NaOH | -- | -- | 1.80 | 1.91 |

| | | | | |
|---|---|---|---|---|
| *Weight percent | | | | |

The catalysts of the invention and those prepared for comparison were tested for activity and selectivity in the reaction for dehydrogenating ethylbenzene to styrene by placing 70 or 100 milliliters (ml) of the above calcined catalyst extrudates in a fixed bed reactor and passing a preheated mixture of steam and ethylbenzene at a weight ratio of 1.5:1 (called the steam to oil ratio) through the bed which was maintained at a temperature of 580 to 590°C. The LHSV (liquid hourly space velocity) was 1.0 and the pressure was maintained at atmospheric. The liquid hourly space velocity was a number denoting residence time in a reactor commonly used by those skilled in the art. After a minimum of 5 days, the weight ratio of steam to ethylbenzene was reduced to 1.2 and the bed temperature adjusted so that an ethylbenzene conversion of 50 percent was achieved. This temperature adjustment was continued each day until a constant conversion of 50 percent was achieved at a fixed bed temperature, that temperature being an indication of the activity of the particular catalyst; that is, the lower the temperature, the higher the activity. The results of the dehydrogenation reaction for Examples 1-5 and Comparative Runs 1-4 are shown in Table 5.

**TABLE 5**

| Catalyst | Temp. °C | Conversion | Selectivity |
|---|---|---|---|
| Example 1 | 589 | 50.0 | 97.2 |
| Example 2 | 597 | 49.8 | 97.1 |
| Example 3 | 589 | 49.7 | 96.9 |
| Example 4 | 596 | 50.3 | 96.5 |
| Example 5 | 600 | 50.5 | 97.2 |
| Comp. Run 1 | 598 | 50.2 | 95.9 |
| Comp. Run 2 | 595 | 50.4 | 96.6 |
| Comp. Run 3 | 590 | 50.4 | 97.1 |
| Comp. Run 4 | 587 | 49.7 | 96.8 |

The moisture resistance of the used catalyst was measured by the following method. After approximately two to three weeks of operation, the catalyst was unloaded from the reactor and twenty randomly chosen extrudates were placed in a glass dish with a flat bottom. The extrudates were separated so that they do not touch each other. The glass dish was then placed in a controlled relative humidity chamber (Vapor-Temp Model No. VP-100AT-1) made by Blue M, a unit of General Signal, adjusted to 30°C and 70 percent relative humidity. After 20 hours the glass dish was removed from the humidity chamber. The excess water in the dish was removed, and the extrudates were placed in a drying oven at 150°C for 24 hours. The extrudates were removed and the average crush strength of the extrudates determined.

Crush strength was a common physical measurement that indicates the strength of the catalyst body, that is, tablet, pellet, or extrudate. In this test, catalyst bodies were compressed between two flat metal surfaces or blocks, and the pressure required to crush the body was measured. A Comten crush strength machine, model no 922T-10-OP, serial no. 830202, was used according to the following procedure. The length of the extrudate was first measured, then the extrudate was crushed between the two blocks of the Comten unit and the pressure required to crush the extrudate was recorded. The crush strength per one-quarter inch length of extrudate was then calculated. This procedure was done on twenty randomly chosen extrudates from each catalyst sample which were all preconditioned as noted in the preceding paragraph. Averaged crush strength was expressed as "PSIG/0.25 inch". The average of these twenty measurements was shown in Table 6. The metric equivalent, kPa/cm, was calculated by multiplying by 10.858 and rounding. The data show the catalysts of this invention after exposure to operating conditions have a superior resistance to physical degradation upon contact with water at temperatures below 100°C than a catalyst prepared in the same manner except for the absence of at least one of the sodium compound or calcium compound, and exposed to substantially the same operating conditions.

**Table 6**

| Catalyst | Average Crush Strength | | Physical Appearance |
|---|---|---|---|
| | PSIG/0.25 inch. | kPa/cm | |
| Example 1 | 18.5 | 201 | Hard, No Cracking or Swelling |
| Example 2 | 18.0 | 195 | Hard, No Cracking or Swelling |
| Example 3 | 13.5 | 147 | Hard, No Cracking or Swelling |
| Example 4 | 15.5 | 168 | Hard, No Cracking or Swelling |
| Example 5 | 18.8 | 204 | Hard, No Cracking or Swelling |
| Comp. Ex. 1 | less than 5 | less than 54 | Soft, Cracked, Swollen |
| Comp. Ex. 2 | less than 5 | less than 54 | Soft, Cracked, Swollen |
| Comp. Ex. 3 | less than 5 | less than 54 | Soft, Cracked, Swollen |
| Comp. Ex. 4 | 6 | 65 | Soft, Cracked, Swollen |

## Claims

1. A calcined dehydrogenation catalyst comprising a calcination product of
(a) at least one iron oxide;
(b) a carbonate, bicarbonate, oxide or hydroxide of potassium or cesium, or a mixture of at least two thereof;
(c) an oxide, carbonate, nitrate, or hydroxide of cerium, or a mixture of at least two thereof;
(d) an hydraulic cement;
(e) a carbonate, sulfate, or hydroxide, of calcium or a mixture of at least two thereof, in an amount sufficient to provide from 1.5 to 20 percent calcium, calculated as calcium oxide, by weight of the calcined catalyst
characterized in that it also comprises:
(f) an hydroxide, carbonate, bicarbonate, acetate, oxalate, nitrate, or sulfate of sodium, in an amount sufficient to provide from 0.2 to 10 percent sodium, calculated as sodium oxide, by weight of the calcined catalyst.

2. The calcined dehydrogenation catalyst of Claim 1 wherein
(f) is sodium hydroxide and (e) is calcium carbonate.

3. The calcined dehydrogenation catalyst of Claim 1 containing from 0.5 to 5 percent sodium calculated as sodium oxide and from 3 to 15 percent calcium calculated as calcium oxide, by weight of the calcined catalyst.

4. The calcined dehydrogenation catalyst of Claim 1 wherein
(b) is a carbonate, bicarbonate, oxide or hydroxide of potassium, or a mixture of at least two thereof.

5. The calcined dehydrogenation catalyst of Claim 4 wherein
(b) is potassium carbonate or a mixture of potassium carbonate and potassium oxide.

6. The calcined dehydrogenation catalyst of Claim 5 wherein
(c) is a carbonate of cerium;
(d) is a calcium aluminate hydraulic cement;
(e) is a carbonate or sulfate of calcium or a mixture thereof, in an amount so that the catalyst contains from 4 to 12 percent by weight calcium, calculated as calcium oxide; and
(f) is sodium hydroxide, in an amount so that the catalyst contains from 0.8 to 3.0 percent by weight sodium, calculated as sodium oxide.

7. The calcined dehydrogenation catalyst of Claim 1 further comprising one or more oxides of chromium, molybdenum, aluminum, vanadium, cobalt, cadmium, copper, magnesium, manganese, or nickel.

8. A process for preparing a calcined dehydrogenation catalyst comprising:
(A) preparing an extrudable mixture by admixing
(a) at least one iron oxide;
(b) a carbonate, bicarbonate, oxide or hydroxide of potassium or cesium, or a mixture of at least two thereof;
(c) an oxide, carbonate, nitrate, or hydroxide of cerium, or a mixture of at least two thereof;
(d) an hydraulic cement;
(e) a carbonate, sulfate, or hydroxide, of calcium or a mixture of at least two thereof, in an amount sufficient to provide from 1.5 to 20 percent calcium, calculated as calcium oxide, by weight of the calcined catalyst,
with sufficient water to form an extrudable mixture;
(B) forming the extrudable mixture into pellets; and
(C) calcining the pellets into a finished catalyst,
characterized in that the catalyst also contains:
(f) an hydroxide, carbonate, bicarbonate, acetate, oxalate, nitrate, or sulfate of sodium, in an amount sufficient to provide from 0.2 to 10 percent sodium, calculated as sodium oxide, by weight of the calcined catalyst.

9. The process of Claim 8 wherein (f) is sodium hydroxide and (e) is calcium carbonate.

10. The process of Claim 8 containing from 0.5 to 5 percent sodium calculated as sodium oxide and from 3 to 15 percent calcium calculated as calcium oxide, by weight of the calcined catalyst.

11. The process of Claim 8 wherein (b) is a carbonate, bicarbonate, oxide or hydroxide of potassium, or a mixture of at least two thereof.

12. The process of Claim 11 wherein (b) is potassium carbonate or a mixture of potassium carbonate and potassium oxide.

13. The process of Claim 12 wherein
(c) is a carbonate of cerium;
(d) is a calcium aluminate hydraulic cement;
(e) is a carbonate or sulfate of calcium or a mixture thereof, in an amount so that the catalyst contains from 4 to 12 percent by weight calcium, calculated as calcium oxide; and the finished catalyst has a superior resistance to physical degradation upon contact with water at temperatures below 100°C than a catalyst prepared in the same manner except that the conditions of at least one of (e) and (f) were not met, and
(f) is sodium hydroxide, in an amount so that the catalyst contains from 0.8 to 3.0 percent by weight sodium, calculated as sodium oxide.

14. The process of Claim 8 wherein one or more compounds of chromium, molybdenum, aluminum, vanadium, cobalt, cadmium, copper, magnesium, manganese, or nickel, as the respective oxide or calcinable to the respective oxide, is included in the extrudable mixture prepared in step (A).

15. A process of dehydrogenating an alkyl aromatic compound to form a vinyl aromatic compound by contacting the alkyl aromatic compound with steam in the presence of a dehydrogenation catalyst which is a catalytically active product of calcining a mixture including
(a) at least one iron oxide,
(b) a carbonate, bicarbonate, oxide or hydroxide of potassium or cesium, or a mixture of at least two thereof,
(c) an oxide, carbonate, nitrate, or hydroxide of cerium, or a mixture of at least two thereof, and
(d) an hydraulic cement, characterized by employing as the dehydrogenation catalyst, the catalytically active product of calcining a mixture which includes, in addition to components (a) through (d):
(e) a carbonate, sulfate, or hydroxide, of calcium or a mixture of at least two thereof, in an amount sufficient to provide from 1.5 to 20 percent calcium, calculated as calcium oxide, by weight of the calcined catalyst; and
(f) an hydroxide carbonate, bicarbonate, acetate, oxalate, nitrate, or sulfate of sodium, in an amount sufficient to provide from 0.2 to 10 percent sodium calculated as sodium oxide, by weight of the calcined catalyst.

16. The process of Claim 15 wherein (f) is sodium hydroxide and (e) is calcium carbonate.

17. The process of Claim 15 wherein the catalyst contains from 0.5 to 5 percent sodium calculated as sodium oxide and from 3 to 15 percent calcium calculated as calcium oxide, by weight of the calcined catalyst.

18. The process of Claim 15 wherein (b) is a carbonate, bicarbonate, oxide or hydroxide of potassium, or a mixture of at least two thereof.

19. The process of Claim 18 wherein (b) is potassium carbonate or a mixture of potassium carbonate and potassium oxide.

20. The process of Claim 19 wherein
(c) is a carbonate of cerium;
(d) is a calcium aluminate hydraulic cement;
(e) is a carbonate or sulfate of calcium or a mixture thereof, in an amount so that the catalyst contains from 4 to 12 percent by weight calcium, calculated as calcium oxide; and
(f) is sodium hydroxide, in an amount so that the catalyst contains from 0.8 to 3.0 percent by weight sodium, calculated as sodium oxide.

21. The process of Claim 20 wherein one or more compounds of chromium, molybdenum, aluminum, vanadium, cobalt, cadmium, copper, magnesium, manganese, or nickel, as the respective oxide or calcinable to the respective oxide, is included in the mixture calcined to form the catalyst.

22. The process of any one of Claim 15 through 19 wherein the alkyl aromatic compound is ethylbenzene and the vinyl aromatic compound is styrene.

23. The process of any one of Claim 15 through 22 wherein the catalyst is reused after both (i) prior exposure of the catalyst to hydrogen and steam at dehydrogenation reaction conditions and (ii) subsequent to such prior exposure, contact of the catalyst with water at a temperature below 100°C.

## Patentansprüche

1. Calcinierter Dehydrierungskatalysator, umfassend ein Calcinierungsprodukt von
(a) wenigstens einem Eisenoxid;
(b) einem Carbonat, Bicarbonat, Oxid oder Hydroxid von Kalium oder Cäsium, oder einer Mischung von wenigstens zwei hiervon;
(c) einem Oxid, Carbonat, Nitrat oder Hydroxid von Cer, oder einer Mischung von wenigstens zwei hiervon;
(d) einem hydraulischen Zement;
(e) einem Carbonat, Sulfat oder Hydroxid von Calcium, oder einer Mischung von wenigstens zwei hiervon in einer ausreichenden Menge, um von 1,5 bis 20 % Calcium, berechnet als Calciumoxid, in Gewicht des calcinierten Katalysators bereitzustellen;
dadurch gekennzeichnet, daß er ebenfalls umfaßt:
(f) ein Hydroxid, Carbonat, Bicarbonat, Acetat, Oxalat, Nitrat oder Sulfat von Natrium in einer ausreichenden Menge, um von 0,2 bis 10 % Natrium, berechnet als Natriumoxid, in Gewicht des calcinierten Katalysators bereitzustellen.

2. Calcinierter Dehydrierungskatalysator nach Anspruch 1, worin (f) Natriumhydroxid ist und (e) Calciumcarbonat ist.

3. Calcinierter Dehydrierungskatalysator nach Anspruch 1, enthaltend von 0,5 bis 5 % Natrium, berechnet als Natriumoxid, und von 3 bis 15 % Calcium, berechnet als Calciumoxid, in Gewicht des calcinierten Katalysators.

4. Calcinierter Dehydrierungskatalysator nach Anspruch 1, worin (b) ein Carbonat, Bicarbonat, Oxid oder Hydroxid von Kalium oder eine Mischung von wenigstens zwei hiervon ist.

5. Calcinierter Dehydrierungskatalysator nach Anspruch 4, worin (b) Kaliumcarbonat oder eine Mischung von Kaliumcarbonat und Kaliumoxid ist.

6. Calcinierter Dehydrierungskatalysator nach Anspruch 5, worin
(c) ein Carbonat von Cer ist;
(d) ein hydraulischer Calcium-Aluminat-Zement ist;
(e) ein Carbonat oder Sulfat von Calcium oder eine Mischung hiervon in einer solchen Menge ist, daß der Katalysator von 4 bis 12 Gew.-% Calcium, berechnet als Calciumoxid, enthält; und
(f) Natriumhydroxid in einer solchen Menge ist, daß der Katalysator von 0,8 bis 3,0 Gew.-% Natrium, berechnet als Natriumoxid, enthält.

7. Calcinierter Dehydrierungskatalysator nach Anspruch 1, weiter enthaltend ein oder mehrere Oxide von Chrom, Molybdän, Aluminium, Vanadium, Cobalt, Cadmium, Kupfer, Magnesium, Mangan oder Nickel.

8. Verfahren zur Herstellung eines calcinerten Dehydrierungskatalysators, umfassend:
(A) Herstellung einer extrudierbaren Mischung durch Zusammenmischen von
(a) wenigstens einem Eisenoxid;
(b) einem Carbonat, Bicarbonat, Oxid oder Hydroxid von Kalium oder Cäsium, oder einer Mischung von wenigstens zwei hiervon;
(c) einem Oxid, Carbonat, Nitrat oder Hydroxid von Cer, oder einer Mischung von wenigstens zwei hiervon;
(d) einem hydraulischen Zement;
(e) einem Carbonat, Sulfat oder Hydroxid von Calcium, oder einer Mischung von wenigstens zwei hiervon in einer ausreichenden Menge, um von 1,5 bis 20 % Calcium, berechnet als Calciumoxid, in Gewicht des calcinierten Katalysators bereitzustellen;
mit ausreichend Wasser zur Bildung einer extrudierbaren Mischung;
(B) Formen der extrudierbaren Mischung in Pellets; und
(C) Calcinieren der Pellets zu einem fertigen Katalysator, dadurch gekennzeichnet, daß der Katalysator ebenfalls enthält:
(f) ein Hydroxid, Carbonat, Bicarbonat, Acetat, Oxalat, Nitrat oder Sulfat von Natrium in einer ausreichenden Menge, um von 0,2 bis 10 % Natrium, berechnet als Natriumoxid, in Gewicht des calcinierten Katalysators bereitzustellen.

9. Verfahren nach Anspruch 8, worin (f) Natriumhydroxid ist und (e) Calciumcarbonat ist.

10. Verfahren nach Anspruch 8, enthaltend von 0,5 bis 5 % Natrium, berechnet als Natriumoxid, und von 3 bis 15 % Calcium, berechnet als Calciumoxid, in Gewicht des calcinierten Katalysators.

11. Verfahren nach Anspruch 8, worin (b) ein Carbonat, Bicarbonat, Oxid oder Hydroxid von Kalium, oder eine Mischung von wenigstens zwei hiervon ist.

12. Verfahren nach Anspruch 11, worin (b) Kaliumcarbonat oder eine Mischung von Kaliumcarbonat und Kaliumoxid ist.

13. Verfahren nach Anspruch 12, worin
(c) ein Carbonat von Cer ist;
(d) ein hydraulischer Calcium-Aluminat-Zement ist;
(e) ein Carbonat oder Sulfat von Calcium oder eine Mischung hiervon in einer solchen Menge ist, daß der Katalysator von 4 bis 12 Gew.-% Calcium, berechnet als Calciumoxid, enthält, und der fertige Katalysator einer überlegenere Festigkeit gegenüber physikalischem Abbau bei Kontakt mit Wasser bei einer Temperatur unterhalb 100° C als ein Katalysator besitzt, der in derselben Weise, ausgenommen, daß die Bedingungen von wenigstens einem (e) oder (f) nicht eingehalten wurden, hergestellt wurde, und
(f) Natriumhydroxid in einer solchen Menge ist, daß der Katalysator von 0,8 bis 3,0 Gew.-% Natrium, berechnet als Natriumoxid, enthält.

14. Verfahren nach Anspruch 8, worin eine oder mehrere Verbindungen von Chrom, Molybdän, Aluminium, Vanadium, Cobalt, Cadmium, Kupfer, Magnesium, Mangan oder Nickel als jeweiliges Oxid oder zu dem entsprechenden Oxid calcinierbar in die in Stufe (A) hergestellte extrudierbare Mischung eingeschlossen werden.

15. Verfahren zum Dehydrieren einer alkylaromatischen Verbindung zur Bildung einer vinylaromatischen Verbindung durch Inkontaktbringen der alkylaromatischen Verbindung mit Dampf in Anwesenheit eines Dehydrierungskatalysators, welcher ein katalytisch aktives Produkt des Calcinierens einer Mischung ist, die einschließt:
(a) wenigstens ein Eisenoxid;
(b) ein Carbonat, Bicarbonat, Oxid oder Hydroxid von Kalium oder Cäsium, oder eine Mischung von wenigstens zwei hiervon;
(c) ein Oxid, Carbonat, Nitrat oder Hydroxid von Cer, oder eine Mischung von wenigstens zwei hiervon;
(d) einen hydraulischen Zement;
dadurch gekennzeichnet, daß als Dehydrierungskatalysator das katalytisch aktive Produkt des Calcinierens einer Mischung verwendet wird, welche zusätzlich zu den Komponenten (a) bis (d) einschließt:
(e) ein Carbonat, Sulfat oder Hydroxid von Calcium, oder eine Mischung von wenigstens zwei hiervon in einer ausreichenden Menge, um von 1,5 bis 20 % Calcium, berechnet als Calciumoxid, in Gewicht des calcinierten Katalysators bereitzustellen; und
(f) ein Hydroxid, Carbonat, Bicarbonat, Acetat, Oxalat, Nitrat oder Sulfat von Natrium in einer ausreichenden Menge, um von 0,2 bis 10 % Natrium, berechnet als Natriumoxid, in Gewicht des calcinierten Katalysators bereitzustellen;

16. Verfahren nach Anspruch 15, worin (f) Natriumhydroxid ist und (e) Calciumcarbonat ist.

17. Verfahren nach Anspruch 15, worin der Katalysator von 0,5 bis 5 % Natrium, berechnet als Natriumoxid, und von 3 bis 15 % Calcium, berechnet als Calciumoxid, in Gewicht des calcinierten Katalysators, enthält.

18. Verfahren nach Anspruch 15, worin (b) ein Carbonat, Bicarbonat, Oxid oder Hydroxid von Kalium, oder eine Mischung von wenigstens zwei hiervon ist.

19. Verfahren nach Anspruch 18, worin (b) Kaliumcarbonat oder eine Mischung von Kaliumcarbonat und Kaliumoxid ist.

20. Verfahren nach Anspruch 19, worin
(c) ein Carbonat von Cer ist;
(d) ein hydraulischer Calcium-Aluminat-Zement ist;
(e) ein Carbonat oder Sulfat von Calcium oder eine Mischung hiervon in einer solchen Menge ist, daß der Katalysator von 4 bis 12 Gew.-% Calcium, berechnet als Calciumoxid, enthält; und
(f) Natriumhydroxid in einer solchen Menge ist, daß der Katalysator von 0,8 bis 3,0 Gew.-% Natrium, berechnet als Natriumoxid, enthält.

21. Verfahren nach Anspruch 20, worin eine oder mehrere Verbindungen von Chrom, Molybdän, Aluminium, Vanadium, Cobalt, Cadmium, Kupfer, Magnesium, Mangan oder Nickel als jeweiliges Oxid oder zu dem entsprechenden Oxid calcinierbar in die zur Bildung des Katalysators calcinierte Mischung eingeschlossen werden.

22. Verfahren nach einem der Ansprüche 15 bis 19, worin die alkylaromatische Verbindung Ethylbenzol ist und die vinylaromatische Verbindung Styrol ist.

23. Verfahren nach einem der Ansprüche 15 bis 22, worin der Katalysator wiederverwendet wird nach sowohl (i) vorheriger Exposition des Katalysators gegenüber Wasserstoff und Dampf bei Dehydrierungs-Reaktionsbedingungen als auch (ii) anschließend an solche vorherige Exposition, Kontakt des Katalysators mit Wasser bei einer Temperatur unterhalb 100° C.

## Revendications

1. Catalyseur calciné de déshydrogénation, comportant le produit de calcination de
a) au moins un oxyde de fer,
b) du carbonate, bicarbonate, oxyde ou hydroxyde de potassium ou de césium ou un mélange d'au moins deux de ces corps,
c) du carbonate, nitrate, oxyde ou hydroxyde de cérium, ou un mélange d'au moins deux de ces corps,
d) un ciment hydraulique, et
e) du carbonate, sulfate ou hydroxyde de calcium, ou un mélange d'au moins deux de ces corps, en une quantité suffisante pour fournir de 1,5 à 20 % de calcium, pourcentage calculé en oxyde de calcium, par rapport au poids du catalyseur calciné,
caractérisé en ce qu'il comporte en outre
f) du carbonate, hydroxyde, bicarbonate, acétate, oxalate, nitrate ou sulfate de sodium, en une quantité suffisante pour fournir de 0,2 à 10 % de sodium, pourcentage calculé en oxyde de sodium, par rapport au poids du catalyseur calciné.

2. Catalyseur calciné de déshydrogénation, conforme à la revendication 1, dans lequel le composant (f) est de l'hydroxyde de sodium et le composant (e) est du carbonate de calcium.

3. Catalyseur calciné de déshydrogénation, conforme à la revendication 1, qui contient de 0,5 à 5 % de sodium, pourcentage calculé en oxyde de sodium, et de 3 à 15 % de calcium pourcentage calculé en oxyde de calcium, par rapport au poids du catalyseur calciné.

4. Catalyseur calciné de déshydrogénation, conforme à la revendication 1, dans lequel le composant (b) est du carbonate, bicarbonate, oxyde ou hydroxyde de potassium, ou un mélange d'au moins deux de ces corps.

5. Catalyseur calciné de déshydrogénation, conforme à la revendication 4, dans lequel le composant (b) est du carbonate de potassium, ou un mélange de carbonate de potassium et d'oxyde de potassium.

6. Catalyseur calciné de déshydrogénation, conforme à la revendication 5, dans lequel
le composant (c) est du carbonate de cérium,
le composant (d) est un ciment hydraulique d'aluminate de calcium,
le composant (f) est de l'hydroxyde de sodium, présent en une quantité telle que le catalyseur contient de 0,8 à 3,0 % en poids de sodium, pourcentage calculé en oxyde de sodium, et
le composant (e) est du carbonate ou du sulfate de calcium, ou un mélange de ces corps, présent en une quantité telle que le catalyseur contient de 4 à 12 % en poids de calcium, pourcentage calculé en oxyde de calcium.

7. Catalyseur calciné de déshydrogénation, conforme à la revendication 1, qui contient encore un ou plusieurs oxydes de chrome, molybdène, aluminium, vanadium, cobalt, cadmium, cuivre, magnésium, manganèse ou nickel.

8. Procédé de préparation d'un catalyseur calciné de déshydrogénation, comportant :
A) le fait de préparer un mélange extrudable, en mélangeant
a) au moins un oxyde de fer,
b) du carbonate, bicarbonate, oxyde ou hydroxyde de potassium ou de césium ou un mélange d'au moins deux de ces corps,
c) du carbonate, nitrate, oxyde ou hydroxyde de cérium, ou un mélange d'au moins deux de ces corps,
d) un ciment hydraulique, et
e) du carbonate, sulfate ou hydroxyde de calcium, ou un mélange d'au moins deux de ces corps, en une quantité suffisante pour fournir de 1,5 à 20 % de calcium, pourcentage calculé en oxyde de calcium, par rapport au poids du catalyseur calciné,
avec assez d'eau pour obtenir un mélange extrudable ;
B) le fait de mettre ce mélange extrudable sous forme de pastilles ; et
C) le fait de calciner ces pastilles pour obtenir un catalyseur fini, caractérisé en ce que le catalyseur contient en outre
f) du carbonate, hydroxyde, bicarbonate, acétate, oxalate, nitrate ou sulfate de sodium, en une quantité suffisante pour fournir de 0,2 à 10 % de sodium, pourcentage calculé en oxyde de sodium, par rapport au poids du catalyseur calciné.

9. Procédé conforme à la revendication 8, dans lequel le composant (f) est de l'hydroxyde de sodium et le composant (e) est du carbonate de calcium.

10. Procédé conforme à la revendication 8, dans lequel le catalyseur contient de 0,5 à 5 % de sodium, pourcentage calculé en oxyde de sodium, et de 3 à 15 % de calcium, pourcentage calculé en oxyde de calcium, par rapport au poids du catalyseur calciné.

11. Procédé conforme à la revendication 8, dans lequel le composant (b) est du carbonate, bicarbonate, oxyde ou hydroxyde de potassium, ou un mélange d'au moins deux de ces corps.

12. Procédé conforme à la revendication 11, dans lequel le composant (b) est du carbonate de potassium, ou un mélange de carbonate de potassium et d'oxyde de potassium.

13. Procédé conforme à la revendication 12, dans lequel
le composant (c) est du carbonate de cérium,
le composant (d) est un ciment hydraulique d'aluminate de calcium,
le composant (f) est de l'hydroxyde de sodium, présent en une quantité telle que le catalyseur contient de 0,8 à 3,0 % en poids de sodium, pourcentage calculé en oxyde de sodium, et
le composant (e) est du carbonate ou du sulfate de calcium, ou un mélange de ces corps, présent en vue quantité telle que le catalyseur contient de 4 à 12 % en poids de calcium, pourcentage calculé en oxyde de calcium ;
et le catalyseur fini, après avoir été mis en contact avec de l'eau à des températures inférieures à 100°C, résiste mieux à la dégradation physique qu'un catalyseur préparé de manière identique à ceci près que ne sont pas remplies les conditions imposées à l'un au moins des composants (e) et (f).

14. Procédé conforme à la revendication 8, dans lequel le mélange extrudable préparé au cours de l'étape (A) contient un ou plusieurs composés de chrome, molybdène, aluminium, vanadium, cobalt, cadmium, cuivre, magnésium, manganèse ou nickel, qui sont les oxydes respectifs ou des composés qui, par calcination, donnent les oxydes respectifs.

15. Procédé de déshydrogénation d'un composé alkyl-aromatique en un composé vinyl-aromatique, dans lequel on met le composé alkyl-aromatique en contact avec de la vapeur d'eau, eu présence d'un catalyseur de déshydrogénation qui est le produit, catalytiquement actif, de la calcination d'un mélange comportant :
a) au moins un oxyde de fer,
b) du carbonate, bicarbonate, oxyde ou hydroxyde de potassium ou de césium ou un mélange d'au moins deux de ces corps,
c) du carbonate, nitrate, oxyde on hydroxyde de cérium, ou un mélange d'au moins deux de ces corps, et
d) un ciment hydraulique,
caractérisé eu ce que l'on emploie, comme catalyseur de déshydrogénation, le produit catalytiquement actif de la calcination d'un mélange qui comporte, en plus des composants (a) à (d) :
e) Du carbonate, sulfate ou hydroxide de calcium, ou un mélange d'au moins deux de ces corps, en une quantité suffisante pour fournier de 1,5 à 20 % de calcium, pourcentage calculé en oxyde de calcium, per rapport au poids du catalyseur calciné, et
f) du carbonate, hydroxyde, bicarbonate, acétate, oxalate, nitrate ou sulfate de sodium, en une quantité suffisante pout fournir de 0,2 à 10 % de sodium, pourcentage calculé en oxyde de sodium, par rapport au poids du catalyseur calciné.

16. Procédé conforme à la revendication 15, dans lequel le composant (f) est de l'hydroxyde de sodium et le composant (e) est du carbonate de calcium.

17. Procédé conforme à la revendication 15, dans lequel le catalyseur contient de 0,5 à 5 % de sodium, pourcentage calculé en oxyde de sodium, et de 3 à 15 % de calcium, pourcentage calculé en oxyde de calcium, par rapport au poids du catalyseur calciné.

18. Procédé conforme à la revendication 15, dans lequel le composant (b) est du carbonate, bicarbonate, oxyde ou hydroxyde de potassium, ou un mélange d'au moins deux de ces corps.

19. Procédé conforme à la revendication 18, dans lequel le composant (b) est du carbonate de potassium, ou un mélange de carbonate de potassium et d'oxyde de potassium.

20. Procédé conforme à la revendication 19, dans lequel
le composant (c) est du carbonate de cérium,
le composant (d) est un ciment hydraulique d'aluminate de calcium,
le composant (e) est de l'hydroxyde de sodium, présent en une quantité telle que le catalyseur contient de 0,8 à 3,0 % en poids de sodium, pourcentage calculé en oxyde de sodium, et
le composant (f) est du carbonate ou du sulfate de calcium, ou un mélange de ces corps, présent en une quantité telle que le catalyseur contient de 4 à 12 % en poids de calcium, pourcentage calculé en oxyde de calcium.

21. Procédé conforme à la revendication 20, dans lequel le mélange qu'on calcine pour obtenir le catalyseur contient un ou plusieurs composés de chrome, molybdène, aluminium, vanadium, cobalt, cadmium, cuivre, magnésium, manganèse ou nickel, qui sont les oxydes respectifs ou des composés qui, par calcination, donnent les oxydes respectifs.

22. Procédé conforme à l'une des revendications 15 à 19, dans lequel le composé alkyl-aromatique est de l'éthylbenzène et le composé vinyl-aromatique est du styrène.

23. Procédé conforme à l'une des revendications 15 à 22, dans lequel on réutilise le catalyseur après les deux opérations suivantes :
i) exposition préalable du catalyseur à de l'hydrogène et de la vapeur d'eau, dans les conditions de la réaction de déshydrogénation, et
ii) à la suite de cette exposition préalable, mise du catalyseur en contact avec de l'eau, à une température inférieure à 100°C.
